# EUROPEAN PATENT SPECIFICATION

(11) **EP 1 046 709 B1**
(45) Date of publication and mention of the grant of the patent: **18.01.2006**
(21) Application number: 99303130.1
(22) Date of filing: 22.04.1999
(51) Int. Cl.: C12N 15/61, C12N 9/90, C12N 15/29, C12N 15/63, C12N 5/10, C12P 5/02

(54) **Rubber producing method using isopentenyl diphosphate isomerase from Hevea Brasiliensis**
Kautschukherstellungsverfahren unter Benutzung von Isopentenyldiphosphateisomerase von Hevea Brasiliensis
Procédé de fabrication de caoutchouc utilisant l'isopentenyl diphosphate isomérase d'Hevea Brasiliensis

(43) Date of publication of application: 25.10.2000
(73) Proprietor: Korea Kumho Petrochemical Co. Ltd., Chongno-Gu, Seoul (KR)
(72) Inventor: Han, Kyung-Whan c/o Dept. of Forestry, East Lansing MI 488240122 (US); Kang, Hun-Seung, Puk-gu, Kwangju-city (KR); Oh, Soo-Kyung, Pundang-gu, Seangnam-city, Kyunggi-do (KR); Shin, Dong-Ho, Kwangsan-gu, Kwangju-city (KR); Yang, Jae-Mo, Puk-gu, Kwangju-city (KR)
(74) Representative: Nash, David Allan

(56) References cited:
- WO-A-97/36998
- US-A- 4 983 729
- JUNG ET AL.: "Camptotheca acuminata isopentenyl diphosphate isomerase I (IPI1) mRNA, complete cds" EMBL SEQUENCE DATABASE, 7 January 1998 (1998-01-07), XP002115458 HEIDELBERG DE -& JUNG ET AL.: "Isopentenyl diphosphate isomerase I" EMBL SEQUENCE DATABASE, 1 June 1998 (1998-06-01), XP002115459 HEIDELBERG DE
- OH ET AL.: "Hevea brasiliensis isopentenyl pyrophosphate isomerase (IPI2) mRNA, complete cds" EMBL SEQUENCE DATABASE, 1 July 1999 (1999-07-01), XP002115460 HEIDELBERG DE -& OH ET AL.: "Hevea brasiliensis isopentenyl pyrophosphate isomerase (IPI1) mRNA, complete cds" EMBL SEQUENCE DATABASE, 1 July 1999 (1999-07-01), XP002115461 HEIDELBERG DE
- KOYAMA ET AL: 'Isopentenyl diphosphate isomerase in rubber latex' PHYTOCHEMISTRY vol. 43, no. 4, 1996, pages 769 - 772

### (SEQUENCE LISTING)

<110> Korea Kumho Petrochemical Co. Ltd.
<120> Isopentenyl diphosphate isomerase from Hevea Brasiliensis
<130> PX99104/EP
<160> 2
<170> KOPATIN 1.0
<210> 1
   <211> 1288
   <212> DNA
   <213> IPIHb (IPP isomerase from Hevea brasiliensis)
<220>
   <221> CDS
   <222> (209)..(910)
<400> 1
   caccactcgc cttctaaatg cccacgccac caccagactc tcgtcctcgc ttccctcctc        60
   tgcttctcct cgttattctt actttctctc tacccaattt gcctctcctt ctctcattca       120
   attccctcta actcttaaac cttcgtctac ctcttcgttc tctagggtat tttcgtcttc       180
   tccatctgca atcaccgcta cttccacc  atg ggt gag gct cca gat gtc ggc         232
                                   Met Gly Glu Ala Pro Asp Val Gly
                                    1               5
   atg gat gct gtc cag aaa cgc ctc atg ttc gac gat gaa tgc att tta         280
   Met Asp Ala Val Gln Lys Arg Leu Met Phe Asp Asp Glu Cys Ile Leu
       10                  15                  20
   gta gat gag aac gat ggt gtt gtt ggt cat gct tcc aaa tat aat tgt         328
   Val Asp Glu Asn Asp Gly Val Val Gly His Ala Ser Lys Tyr Asn Cys
   25                  30                  35                  40
   cat ttg tgg gaa aat att ttg aag ggg aac gca tta cat aga gct ttt         376
   His Leu Trp Glu Asn Ile Leu Lys Gly Asn Ala Leu His Arg Ala Phe
                   45                  50                  55
   agc gta ttt ctc ttc aac tca aaa tat gag cta ctc ctt cag caa cgc         424
   Ser Val Phe Leu Phe Asn Ser Lys Tyr Glu Leu Leu Leu Gln Gln Arg
               60                  65                  70
   tct ggg aca aag gtg aca ttc ccg ctt gta tgg aca aac act tgc tgt         472
   Ser Gly Thr Lys Val Thr Phe Pro Leu Val Trp Thr Asn Thr Cys Cys
           75                  80                  85
   agt cat cct ctg tac cgt gaa tct gag ctt att gat gag gat gct ctt         520
   Ser His Pro Leu Tyr Arg Glu Ser Glu Leu Ile Asp Glu Asp Ala Leu
           90                  95                  100
   ggt gtg aga aat gct gca caa agg aag ctt ttc gat gag ctt ggt atc         568
   Gly Val Arg Asn Ala Ala Gln Arg Lys Leu Phe Asp Glu Leu Gly Ile
   105                 110                 115                 120
   cct gct gaa gat gtt cca gtt gat cag ttt act cca cta gga cgt ata         616
   Pro Ala Glu Asp Val Pro Val Asp Gln Phe Thr Pro Leu Gly Arg Ile
                   125                 130                 135
   cta tat aag gcg tcc tcc gat gga aag tgg gga gag cat gaa ctt gat         664
   Leu Tyr Lys Ala Ser Ser Asp Gly Lys Trp Gly Glu His Glu Leu Asp
               140                 145                 150
   tat ctg ctc ttt ata gtc cgt gat gtt aat gta aat cca aac cct gat         712
   Tyr Leu Leu Phe Ile Val Arg Asp Val Asn Val Asn Pro Asn Pro Asp
           155                 160                 165
   gag gta gct gat gta aag tat gtt aac cgg gat cag ttg aag gag ctc         760
   Glu Val Ala Asp Val Lys Tyr Val Asn Arg Asp Gln Leu Lys Glu Leu
       170                 175                 180
   ttg agg aag gcg gat tct ggc gag gaa ggt ata aat ttg tca cct tgg         808
   Leu Arg Lys Ala Asp Ser Gly Glu Glu Gly Ile Asn Leu Ser Pro Trp
   185                 190                 195                 200
   ttt aga cta gtt gtg gac aac ttc ttg ttg aaa tgg tgg gaa aat gtc         856
   Phe Arg Leu Val Val Asp Asn Phe Leu Leu Lys Trp Trp Glu Asn Val
                   205                 210                 215
   gaa aat ggg aca ctc aag gaa gca gtt gac atg aaa acg att cac aag         904
   Glu Asn Gly Thr Leu Lys Glu Ala Val Asp Met Lys Thr Ile His Lys
               220                 225                 230
   ttg agt   tgagataaat atctatacag aatcaaattt gattgtggca tcacctgtag       960
   Leu Ser
   ctttattatt tgaataaatt tggtaatgtc agttgtaaac gagttagcct ttctttgctg      1020
   ttgtggctgt tagcctggtc caaagttaca ttaaattttt tgatgtttcc tgaaagcagc      1080
   tgggcagttc tgatattgta acttagattt ggctcccatc atttaaaact gaataaggag      1140
   attttcactt cctttatatc cttaaattgg aggaattttt ttgtctcccc ttgttgagat      1200
   cttttttcgg ggtgaggaca ttgttatttg ttttgttatg aatcaagtga gactggctat     1260
   tcaagcaaaa aaaaaaaaaa aaaaaaaa                                          1288
<210> 2
   <211> 234
   <212> PRT
   <213> IPIHb (IPP isomerase from Hevea brasiliensis)
<400> 2
   Met Gly Glu Ala Pro Asp Val Gly Met Asp Ala Val Gln Lys Arg Leu
   1               5                   10                  15
   Met Phe Asp Asp Glu Cys Ile Leu Val Asp Glu Asn Asp Gly Val Val
               20                  25                  30
   Gly His Ala Ser Lys Tyr Asn Cys His Leu Trp Glu Asn Ile Leu Lys
           35                  40                  45
   Gly Asn Ala Leu His Arg Ala Phe Ser Val Phe Leu Phe Asn Ser Lys
       50                  55                  60
   Tyr Glu Leu Leu Leu Gln Gln Arg Ser Gly Thr Lys Val Thr Phe Pro
   65                  70                  75                  80
   Leu Val Trp Thr Asn Thr Cys Cys Ser His Pro Leu Tyr Arg Glu Ser
                   85                  90                  95
   Glu Leu Ile Asp Glu Asp Ala Leu Gly Val Arg Asn Ala Ala Gln Arg
               100                 105                 110
   Lys Leu Phe Asp Glu Leu Gly Ile Pro Ala Glu Asp Val Pro Val Asp
           115                 120                 125
   Gln Phe Thr Pro Leu Gly Arg Ile Leu Tyr Lys Ala Ser Ser Asp Gly
       130                 135                 140
   Lys Trp Gly Glu His Glu Leu Asp Tyr Leu Leu Phe Ile Val Arg Asp
   145                 150                 155                 160
   Val Asn Val Asn Pro Asn Pro Asp Glu Val Ala Asp Val Lys Tyr Val
                   165                 170                 175
   Asn Arg Asp Gln Leu Lys Glu Leu Leu Arg Lys Ala Asp Ser Gly Glu
               180                 185                 190
   Glu Gly Ile Asn Leu Ser Pro Trp Phe Arg Leu Val Val Asp Asn Phe
           195                 200                 205
   Leu Leu Lys Trp Trp Glu Asn Val Glu Asn Gly Thr Leu Lys Glu Ala
       210                 215                 220
   Val Asp Met Lys Thr Ile His Lys Leu Ser
   225                 230

## Description

### BACKGROUND OF INVENTION

### Field of the Invention

The present invention relates to a isopentenyl diphosphate isomerase from *Hevea Brasiliensis* and a rubber producing method using the same.

### Description of the Prior Art

The isoprenoid biosynthetic pathway is ubiquitous to all living organisms and produces more than 23,000 compounds which play vital roles in the structure of cells, electron transport, photosynthesis, cell-to-cell signaling, and interactions between organisms (Ramos-Valdivia et al., 1997 Nat. Prod. Rep. 14 : 591 - 603). Several important classes of compounds derived from this complex pathway include sterols, carotenoids, dolichols, ubiquinones, and prenylated proteins (Hahn and Poulter, 1995 J. Biol. Chem. 270 : 11298 - 11303). All of these compounds are derived from the same building block, IPP. In most eukaryotes, IPP is synthesized from three molecules of acetyl-CoA *via* the mevalonic acid pathway(Chappell, 1995 Annu. Rev. Plant Physiol. Plant Mol. Biol. 46 : 521 - 547). Recently, a separate mevalonate-independent pathway was discovered in bacteria and plant(Lichtenthaler et al., 1997 FEBS Lett. 400 . 271 - 274; Rohmer et al., 1993 Biochem. Biophys Res. Commun 196:, 1414 - 1421). This pathway utilizes three-carbon precursors such as glyceraldehyde phosphate, pyruvate, and dihydroxyacetone phosphate. The enzyme IPP isomerase (hereafter "IPI") catalyzes the interconversion of IPP to its highly electrophilic isomer, dimethylallyl diphosphate (DMAPP). These two isomers serve as substrates for the synthesis of isoprenoid compounds.

Bioengineering of isoprenoid biosynthesis is of keen interest because of the commercial value of plant compounds such as essential oils, pharmaceuticals, rubber, and waxes see US 4,983,729. Although the interconversion of IPP and DMAPP is catalyzed by the same IPP isomerase, the equilibrium is in favor of DMAPP production(Sun et al., 1998 Proc. Natl. Acad. Sci USA 95 : 11482-11488). This isomerization reaction may be a rate-limiting step for isoprenoid biosynthesis. Expression of an exogenous IPI gene enhanced isoprenoid biosynthesis in *E. coli*(Kajiwara et al., 1997 Biochem J. 324 : 421-426). Transgenic tobacco plants transformed with a *H. brasiliensis* HMGCoA reductase over-produced total sterols up to six-times than the control plants did(Schaller et al., 1995 Prior Physiol. 109 : 761-770).

Natural rubber (*cis*-1,4-polyisoprene) is an important raw material for many industrial uses. Although rubber is produced in about 2,000 plant species(Backhaus, 1985 Israel J. Biol, 34 : 283-293), *H. brasiliensis* has been the only commercial source of natural rubber mainly due to its abundance in the tree, its quality and the ease of harvesting. Diminishing acreage of rubber plantation coupled with increasing demand has renewed research interests on the study of rubber biosynthesis and the development of an alternative rubber source. The first step in rubber biosynthesis is the isomerization of IPP to DMAPP by IPP isomerase. The successive head-to-tail condensation reactions of the five-carbon intermediates catalyzed by enzyme(s) referred to as rubber transferase (or polymerase) have been assumed to yield rubber. IPP isomerase activity has been found in bottom fraction and C-serum of rubber latex of *H. brasiliensis*(Koyama et al., 1996 Phytochemistry 43 : 769-772; Tangpakdee et al., 1997 Phytochemistry 45 : 261-267). The presence of a site-directed specific inhibitor of IPP isomerase, 3,4-oxido-3-methyl-l-butyl diphosphate (OMBPP), inhibited incorporation of IPP into rubber in *in vitro* rubber assay(Cornish, 1993 Eur. J. Biochem. 218 : 267-271).

IPP isomerase catalyzes the conversion of IPP to DMAPP, which is an essential step in the biosynthesis of all isoprenoids including carotenoids, growth regulators, and natural rubber. The enzyme from a wide variety of organisms has been studied for a review, see(Ramos-Valdivia et al., 1997 Nat. Prod. Rep. 14 : 591-603). In higher plants, IPP isomerase has been purified and characterized from *Capsicum* chromoplasts(Dogbo and Camara, 1987 Biochem Biophys. Actu 920 : 140-148), *Cinchona robusta* cell suspension(Ramos-Valdivia et al., 1997 Eur. J. Biochem. 249 : 161-170), *H. brasiliensis*(Koyama et al., 1996 Phytochemistry 43 : 769-772). To date relatively few IPP isomerase sequences from higher plants appear in the literature. Full-length genes for higher plant IPP isomerase are known only for *Arabidopsis*(Campbell et al., 1997 Plant Mol. Biol. 36 : 323-328, WO 97/36998) and two flowering plants *Clarkia* spp.(Blanc et al., 1996 Plant Physiol. 111 : 652; Blanc and Pichersky, 1995 Plant Physiol. 108 : 255-856). Partial sequence is also available for tobacco (Accession No. Y09634). However, none of the genes has been implicated in rubber biosynthesis.

As the isomerization of IPP to DMAPP is the first step in rubber biosynthesis, it is plausible for IPP isomerase to play a key role in rubber biosynthesis. Although IPP isomerase activity was observed in both bottom and C-serum fractions from *Hevea* latex(Tangpakdee et al., 1997 Phytochemistry 45 : 261-267), neither the protein nor cDNA encoding IPP isomerase has been isolated and studied from *Hevea* rubber tree.

### SUMMARY OF THE INVENTION

Accordingly, it is an object of the present invention to provide a rubber producing method using a isopentenyl diphosphate isomerase from *Hevea Brasiliensis*.

The present inventors cloned the gene by screening a latex cDNA library using a PCR-generated probe. Despite the presumed-crucial role of the enzyme in rubber biosynthesis, the gene does not seem to be expressed as an abundant transcript in latex, which contains 30-50% (wt/wt) of *cis*-1,4-polyisoprene (rubber). In a separate study, the present inventors have generated 245 expressed sequence tags (ESTs) to study gene expression profile in the latex of *H. brasiliensis*(Han et al., 1999 Tree Physiol. 20 : 503-540). The gene encoding IPP isomerase was not identified among the ESTs. Furthermore, initial attempts to screen 1 X 10⁶ or less plaques of the latex cDNA library failed to produce any hybridizing cDNA clones. After screening of more than 2 X 10⁶ plaques only two hybridizing clones were obtained. Since DMAPP is the primer for the isoprenoid biosynthesis and must be present in any cell compartment where isoprenoids are biosynthesized, the low transcript level of IPIHb gene in the latex is unexpected.

### BRIEF DESCRIPTION OF THE DRAWINGS

Other objects and aspects of the present invention will become apparent from the following description of embodiments with reference to the accompanying drawings in which:

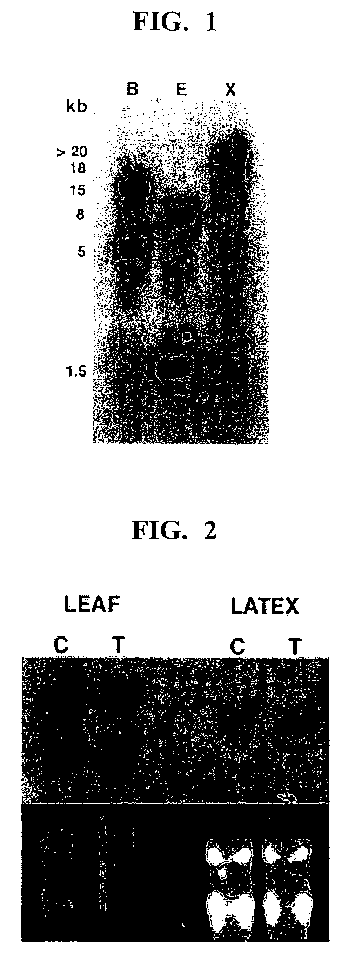

Fig. 1. is Southern blots of genomic DNA from leaves of *H. brasiliensis* probed with internal fragment of IPIHb. Lanes B, E, and X correspond to digestion of total DNA with *Bam*HI, *Eco*RI, and *Xho*I, respectively.
Fig. 2. is Nothern blot analysis of IPIHb transcript. Total RNA isolated from leaf and latex of *H. brasiliensis* were hybridized with a ³²P-labelled internal fragment of IPIHb cDNA. The lower panel shows the ethidium bromide-stained rRNA under UV light before blotting, indicating the loading of similar amount of total RNA. Lanes C and T correspond to untreated control and wounded samples, respectively. For the effect of wounding, five *H. brasiliensis* (clone RRIM600) trees were wounded with six nails above and along the slope of the tapping cut while another five control trees were not punctured. Latex and leaf samples were collected from each tree about 16 h after puncturing.

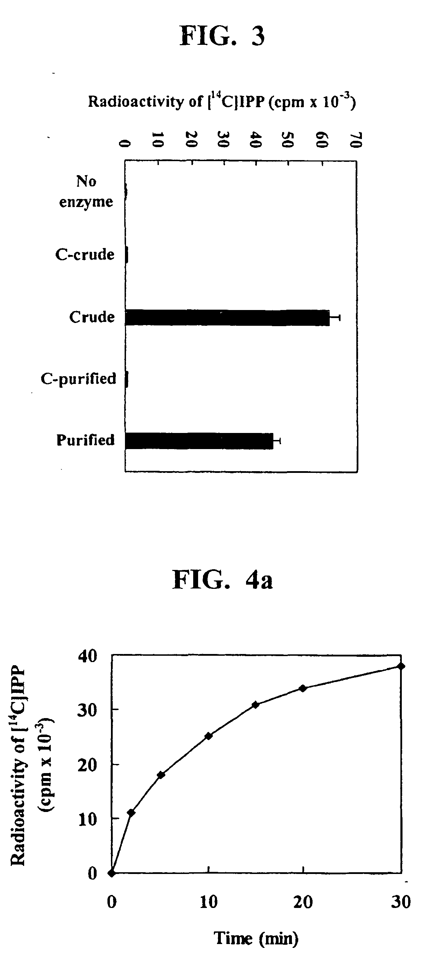

Fig. 3. is Analysis of IPP isomerase activity. IPP isomerase assay was performed in 50 µL of reaction mixture containing 20 µM [¹⁴C]IPP and 5 µL of enzyme preparation, and radioactivity of the acid labile products was measured by a liquid scintillation counter. Each value is the mean of three experiments; No enzyme, without cell culture extracts; C-crude, bacterial extracts of the cells expressing GST-IPIHb fusion protein without IPTG induction; Crude, cell culture extracts with IPTG induction; C-purified, purified GST-IPIHb fusion protein from the cells without IPTG induction; Purified, purified GST-IPIHb fusion protein from the cells with IPTG induction.

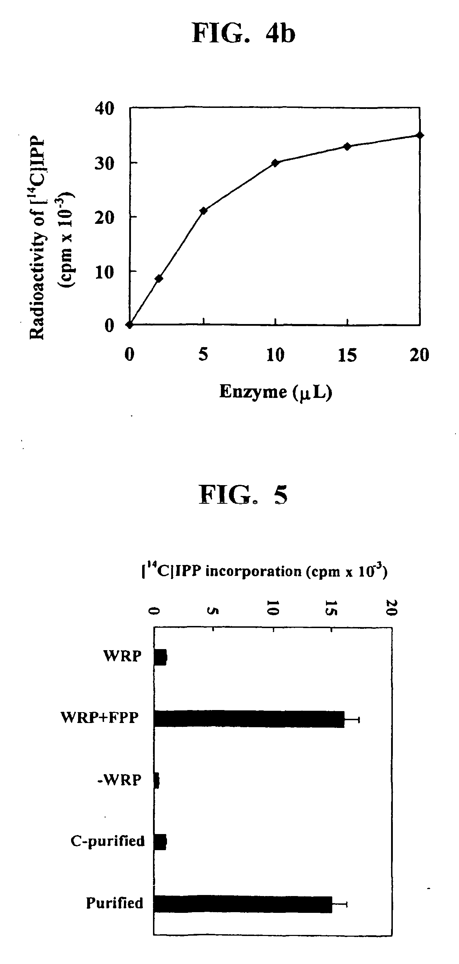

Fig. 4a is Time course and Fig. 4b is concentration dependence of enzymatic activity of IPP isomerase. The IPP isomerase assay was performed in 50 µL of reaction mixture containing 5 µL of purified GST-IPIHb fusion protein for time course experiment, and the reaction was for 10 min with different amounts of enzyme.
Fig. 5. is Effect of IPP isomerase on rubber biosynthesis *in vitro.* Incorporation of [¹⁴C]IPP into growing rubber chain was measured in 50 µL of reaction mixture containing 10 mg of WRP and 5 µL of indicated enzyme preparation, and without FPP, an efficient initiating molecule for rubber biosynthesis. Each value is the mean of three experiments; WRP, rubber assay with WRP alone; WRP+FPP, assay with WRP and FPP; -WRP, assay with purified GST-IPIHb fusion protein and without WRP; C-purified, assay with WRP and purified GST-IPIHb fusion protein from the cells without IPTG induction; Purified, assay with WRP and purified GST-IPIHb fusion protein from the cells with IPTG induction.

### DESCRIPTION OF THE PREFERRED EMBODIMENTS

### Gene isolation

Based on the cDNA sequence of *A. thaliana*(Campbell et al., 1997 Plant Mol. Biol. 36 : 323-328, WO 97/36998), a PCR primer (downstream) was constructed and used with M13 reverse primer (upstream) to amplify the *Hevea* IPP isomerase gene using a latex cDNA library of *H. brasiliensis.* The PCR amplified a partial sequence of IPP isomerase coding region. In an attempt to clone the gene by PCR, two PCR primers (IPP-L1 and IPP-L2) from the sequence information were designed and used in combination with vector (M13 forward and reverse) primers. The PCR reaction with the combination of IPP-L2 (downstream) and M13 reverse (upstream) primers yielded about 700-bp fragment. However, no amplification product was obtained with IPP-L1 (upstream) and M13 forward (downstream) primers. The 700-bp PCR fragment showed high sequence identity to the cDNA sequence of *A. thaliana* and was used as a probe to screen the same cDNA library. A total of 2 X 10⁶ plaques were screened. The plaques hybridized to the probe and were subjected for *in vivo* excision. The resulting phagemids contained a full-length cDNA and the gene was designated IPIHb. The DNA sequence is 1,288 bp and has a continuous open reading frame (ORF) encoding a peptide of 234 amino acids with a predicted molecular mass of 26.7 kDa (Sequence ID No. 1). The deduced protein is acidic with an isoelectric point of 4.7 which is lower than that of IPP isomerase from *A. thaliana* (pI = 6.0).

### Southern analysis indicates two genes for IPP isomerase

To determine the copy number of IPP isomerase gene, Southern blot hybridization analysis was performed using the 700-bp PCR amplified fragment of IPIHb as a hybridization probe. The genomic DNA was digested with *BamH*I, *Eco*RI, and *Xho*I. These enzymes do not have restriction sites inside the coding region of IPIHb. Two hybridizing bands were observed from each restriction digest under both low (55 °C) and high (65 °C) stringency conditions (Fig. 1). These results suggest that IPP isomerase is encoded by multiple genes in *Hevea* rubber tree. This seems to corroborate the observations in the literature. Campbell et al.(Campbell et al., 1997 Plant. Mol. Biol. 36 : 323-328) reported two cDNA clones encoding IPP isomerase and concluded that the two genes are derived from a small gene family. Multiple isoforms of IPP isomerase have been described in *C. breweri*(Blanc et al., 1996 Plant Physiol 111 : 652) and *C. robusta*(Ramos-Valdivia et al., 1997 Eur. J. Biochem 249 : 161-170). Thus, the occurrence of IPI isoforms is a general feature of higher plants, although specific role of individual isoform is unknown. The presence of uninterrupted 71-amino acid stretch in the region immediately upstream of the ORF of IPIHb suggests that there are isoform(s) targeted to subcellular organelles.

### Expression of mRNA for IPIHb

The expression of the IPP isomerase gene in latex and leaf tissues was investigated. Two different sizes of transcripts (1.2 and 0.6 kb) were hybridized to the probe in the blot containing total RNA from leaf tissues, while only one hybridizing band (1.0 kb) was detected in the blot from latex (Fig. 2). To test the inducible nature of IPP isomerase with wound treatment, leaf and latex samples from wound-treated *Hevea* trees were obtained(Oh et al., 1999 J. Biol Chem. 274 : 17132-17138). Analyses of RNA extracted from extruded latex and leaf tissues of the wounded trees showed that the wounding did not change the expression level of IPP isomerase (Fig. 2).

### Functional analysis of IPP isomerase expressed in E. coli

To further characterize the cloned cDNA encoding *Hevea* IPP isomerase, a GST-IPIHb fusion gene to produce pGEX-IPIHb was constructed. The pGEX expression system provides simple purification of fusion proteins from crude bacterial lysates. Expression and purification of the GST-IPIHb fusion protein was monitored by SDS-PAGE (data not shown). Enzymatic activity of IPP isomerase was analyzed using either the crude cell extract or the purified GST-IPIHb fusion protein. As a control, either the same bacterial extracts but without IPTG induction or the culture of *E. coli* harboring pGEX that carries a cDNA coding only the GST protein was used.

The conversion of IPP to DMAPP was confirmed by the increase in radioactivity resulting from acid labile allylic diphosphate in the reaction mixture. As shown in Fig. 3, basal level of radioactivity was observed for the reaction mixture without enzyme preparation. Similar basal level of radioactivity was detected for the reaction mixture containing enzyme preparation of the cells without IPTG induction. In contrast, a remarked increase in radioactivity was observed for the reaction mixture containing either the crude extract or the purified GST-IPIHb fusion protein of the cells with IPTG induction. In a time course experiment using purified GST-IPIHb fusion protein, it was clearly noted that radioactivity from acid-labile ¹⁴C allylic diphosphate increased with incubation time (Fig. 4a). It was also observed that the radioactivity increased with the amount of purified GST-IPIHb fusion protein added in the reaction mixture (Fig. 4b). The time and concentration dependent increases in radioactivity resulting from acid-labile ¹⁴C allylic diphosphate indicate that the conversion of IPP to DMAPP occurred *in vitro* by GST-IPIHb fusion protein. These results reveal that the cDNA clone encodes a functional IPP isomerase as part of GST-IPIHb fusion protein.

### IPP isomerase is required for rubber biosynthesis in vitro

Formation of new rubber chain occurs via condensation of IPP to initiating molecules such as GGPP, FPP, GPP and DMAPP. Therefore, general procedure for analyzing rubber biosynthetic activity *in vitro* contains one of the initiating allylic diphosphates in the reaction mixture. In order to test the role of IPP isomerase on rubber biosynthesis, and confirm further that the cDNA which isolated in the present invention encodes a functional IPP isomerase, *in vitro* rubber biosynthesis assays using WRP deprived of initiating allylic diphosphates were performed with the addition of IPP isomerase in the reaction mixture. It was clearly noted that FPP, an efficient initiating molecule, was required for rubber biosynthesis in the reaction mixture containing WRP (Fig. 5). The reaction mixture containing bacterial extracts of the un-induced *E. coli* cells harboring same cDNA clone for GST-IPIHb fusion protein showed a basal level of [¹⁴C]IPP incorporation. In contrast, a marked increase of [¹⁴C]IPP incorporation into rubber was observed for the reaction mixture containing WRP and purified GST-IPIHb fusion protein (Fig. 5). No incorporation of [¹⁴C]IPP into rubber was observed in the reaction mixture without WRP, indicating that the increase of radioactivity in the reaction mixture containing WRP and GST-IPIHb fusion protein arose from the synthesis of new rubber chain. These results reveal that the GST-IPIHb fusion protein encoded from the cDNA clone is catalytically active in converting IPP to DMAPP, a key activation step of the basic five-carbon isoprene unit in rubber biosynthesis.

A DNA sequence that is capable of encoding a IPP isomerase (hereafter "IPIHb DNA sequence") has been isolated and a hybrid vector and a transformed host, respectively, that contain the IPIHb DNA sequence have been provided.

"IPIHb" refers to the IPP isomerase isolated from *Hevea brasiliensis.* A "hybrid vector" as used herein, refers to a vector formed by ligation of DNA from a plasmid, bacterophage, plant virus or other vectors with DNA or cDNA from *Hevea* or other organisms that are capable of producing IPIHb, such plants or other organisms being collectively referred to herein as "plant materials."

Plant materials from, e.g., *Hevea,* are used to produce a cDNA. A cDNA library is constructed that is based upon mRNA sequences isolated from total RNA from the plant materials. A first strand cDNA can be synthesized enzymatically using the isolated mRNA as a template, an oligo dT sequence as a primer and a reverse transcriptase as the enzyme. After construction of the first strand cDNA, a second strand cDNA can be synthesized enzymatically using the first strand cDNA as a template and a DNA polymerase as the enzyme. The resulting double-stranded cDNA molecules are inserted into a suitable vector, to produce a cDNA library.

The resulting cDNA library may be screened for the full-length sequence of IPIHb gene using a nucleic acid probe specific for the IPIHb gene (hereafter "IPIHb gene specific probe"). The IPIHb gene-containing cell can then be propagated and large amounts of DNA sequence encoding IPIHb can be extracted.

A nucleic acid probe for the identification of IPIHb gene may be produced by PCR amplification of the cDNA library using IPP isomerase specific primers.

Plant cDNA or cDNA from other organisms that hybridizes with a labelled nucleic acid probe specific for the IPIHb gene can be identified and isolated. The isolated DNA can be ligated to a vector DNA to produce a hybrid vector. The hybrid vector can be used to transform a competent host and to induce the production of IPP isomerase in the host.

A vector that is suitable for use can be a plasmid or a virus that is capable of being transferred into a host cell or of infecting a host cell and of replicating in a host cell. A suitable vector is one that is capable of carrying as an insert an entire IPIHb DNA sequence in a non-essential region of the vector DNA.

A suitable transformed host is one that is capable of expressing the IPIHb DNA sequence. Preferably, a suitable transformed host is incapable of catalyzing the conversion of IPP to rubber. Such a host maybe a plant, a bacterium or a fungus. The plant host may be an annual plant such as tobacco species, a grass species or a perennial plant species. The bacterium can be any bacterium, e.g., *E. coli,* a *Bacillus* species or an *Agrobacterium* species. The fungus is selected from the group consisting of a lactarius species and an *Aspergillus* species.

The DNA fragment that contains the IPIHb gene can be ligated to a suitable promoter so as to place the gene under the control of the promoter. A suitable promoter is one that is capable of functioning in a transformed host. For example, if the host to be transformed is a plant, the IPIHb gene can be ligated to a plant promoter such as Pnos, the promoter for nopaline synthetase; if the host to be transformed is a bacterium, the IPIHb gene can be ligated to a bacterial promoter such as the trp or tac promoter of *E. coli.* In the alternative, the IPIHb gene can be ligated to a virus promoter such as the 16S and 35S. promoter of cauliflower mosaic virus.

A cDNA library, instead of a genomic DNA library, is constructed using mRNA isolated from total RNA. Total RNA is obtained from plant materials by a method that is substantially the same as that of Kush et al. Proc Natl Acad Sci U S A *87*, 1787-1790 (1990). The plant materials that are used for the isolation of mRNA can be from any plant or organisms that are capable of synthesizing rubber. For example, since *Hevea* trees produce rubber from latex, mRNA capable of encoding IPIHb can be obtained from *Hevea* latex.

The isolation of mRNA herein capitalizes on the presence of a poly(A) tail at the 3' end of the mRNA. The 3' tail is utilized to separate the mRNA from the other RNA species. Separation is achieved by chromatography on an oligo(dT) cellulose column by binding of the poly(A) tail of mRNA with the T residues on the cellulose column. The unbound RNA can be washed free of the column and the mRNA can be eluted by buffers that destabilize the A-T duplex. The RNA concentration is then determined spectrophotometrically. The isolated mRNA is then used as a template for synthesis of first-strand cDNA molecules. The first-strand cDNA is, in turn, used as a template in a second-strand DNA synthesis utilizing, e.g., according to Sambrook et al., MOLECULAR CLONING, Cold Spring Harbor, N.Y. 1987.

A suitable vector capable of carrying an entire gene in a non-essential region of its DNA and capable of replicating in a host cell, e.g., a lambda vector, can be ligated to the DNA fragments to generate a library of recombinant DNA molecules. *In vitro* packaging of these recombinant molecules can yield an infectious phage stock consisting of recombinant clones. The recombinant DNA libraries constructed in this manner can be used for isolation of the IPIHb gene and other genes that are important in the biosynthesis of rubber utilizing either nucleic acid probes or antibodies if the cDNA library expresses the cDNA.

A nucleic acid probe for the detection of the IPIHb gene can be modified to detect extremely small amounts of the gene, e.g., by labeling the probe with [.sup.³² P]-phosphate at the 3' or 5' end, or with biotin moieties by conventional laboratory techniques as in, e.g., according to Sambrook et al., MOLECULAR CLONING, Cold Spring Harbor, N.Y. 1987. The radioactive labeling can be performed using commercially available 3' or 5' end labeling kits from, e.g., Amersham Corporation (Arlington Heights, III.) or New England Nuclear (Boston, Mass.), and following the manufacturer's directions. The IPIHb gene in the DNA libraries can then be identified by hybridization with the labeled probe.

A recombinant lambda Phage containing *Hevea* cDNA library is screened substantially according to the method of Sambrook et al., loc. cit., using [.sup.³² P]-labelled IPIHb specific oligonucleotide.

The clone that carries the IPIHb gene and that reacts positively with the IPIHb gene specific probe can be isolated and converted into a plasmid by in vivo excision according to the protocol provided by the cDNA library kit. This method of conversion of phage DNA into plasmid is feasible because when the Hevea cDNA library is constructed into the Uni-ZAP II vector (Stratagene, La Jolla, CA) that was designed for such in vivo excision. The plasmid is transferred and maintained in *E. coli* for production of large quantities of this gene. The *E. coli* cells containing the plasmid with the IPIHb gene are propagated to produce large quantities of the IPIHb DNA sequence. This DNA can be extracted from the transformed hosts in accordance with conventional laboratory techniques.

DNA sequences and recombinant DNA molecules coding for the *Hevea* small rubber particle protein (IPIHb) and processes for the production of recombinant protein, to vectors containing the sequences, to cultures producing recombinant protein, and to the materials significant in the production of recombinant protein are presented. A DNA sequence encoding IPIHb or a protein having substantially the same biological activity for rubber biosynthesis as IPIHb is shown.

The isolated recombinant vector containing cDNA encoding the IPIHb is transferable to other prokaryotic or eukaryotic host organisms wherein the IPIHb DNA will be expressed to produce functional IPIHb for rubber biosynthesis.

The following example is given by way of illustration to facilitate a better understanding of the present invention.

### EXAMPLE 1

### Plant materials

Latex and leaf samples were obtained from mature rubber plants (*H. brasiliensis* clone RRIM600) growing at the Rubber Research Institute of Malaysia, Selangor, Malaysia. Latex exuding from the tapped trees was collected while continuously mixing it with an equal volume of 2 X RNA extraction buffer (0.1 M Tris·HCl, 0.3 M LiCl, 0.01 M EDTA, 10% SDS, pH 9.5) either at ambient temperature or on ice(Han et al., 1999). The collected samples were frozen in liquid nitrogen and shipped back to the laboratory on dry ice.

### Total and poly(A)⁺ RNA isolation

RNA extraction from latex was performed as described previously(Oh et al., 1999) using the Qiagen Rneasy Plant Minikit (Qiagen Inc, Chatsworth, CA). Poly(A)⁺ RNA was isolated using Oligotex-dT™ mRNA kit (Qiagen Inc, Chatsworth, CA).

### PCR amplification of H. brasiliensis IPP isomerase (IPIHb)

PCR amplification was performed using latex cDNA library as a template. A PCR primer (IPP-A2) corresponding to the sequences from +606 to +629 bp in *Arabidopsis* IPP isomerase was designed. The primers used to amplify a fragment of the IPIHb cDNA were IPP-A2 (5'-GTAATCAAGTTCATGCTCTCCCCA-3') and M13 reverse primer. PCR was performed for 30 cycles of 30 s at 94 °C, 30 s at 55 °C, and 30 s at 72 °C, with a 5-min pre-heat and a 7-min final extension at 72 °C. The PCR product was excised from an agarose gel and purified with the QIAEX II Agarose Gel Extraction Kit (QIAGEN, Hilden, Germany). Purified PCR products were ligated into the pGEM®-T vector (Promega, Madison, WI). Plasmids were transformed into *E. coli* strain XLI-Blue, isolated, and sequenced. Based on the PCR-amplified sequence of *Hevea* IPP isomerase, two primers were designed, IPP-L1 as downstream primer (5'-TATGAGCTACTCCTTCAGCAACGCTCT-3') and IPP-L2 as downstream primer (5'-ATCAAGTTCATGCTCTCCCCACTTTCC-3'). The IPP-L1 and L2 primers were used with M13 forward and reverse primers, respectively.

### Construction and screening of latex cDNA library

A cDNA library was constructed in a Uni-ZAP II vector according to the supplier's instructions (Stratagene, La Jolla, CA) using poly(A)⁺ RNA prepared from latex as described above. The primer (IPP-L2, downstream) and M13 reverse primer (upstream) were used to amplify partial IPP isomerase fragment from the latex cDNA library. The 700-bp PCR product was used to screen 2 x 10⁶ plaques of the latex cDNA library as described previously(Oh et al., 1999 J. Biol. Chem 274 : 17132-17138). The cDNA clones hybridized to the probe were subjected to *in vivo* excision according to the protocol provided by the cDNA library kit and sequenced. A clone carrying a full-length cDNA insert was isolated and designated as IPIHb.

### Sequencing of cDNA clones and computer analysis

Plasmid DNA for sequencing reactions was prepared by the alkaline lysis method (Sambrook et al., 1989 Molecular cloning; A laboratory manual. 2nd edition) using Wizard® Plus SV Minipreps DNA Purification System kit (Promega, Madison, WI). The sequencing reaction was performed with the ALF Express AutoRead Sequencing kit (Pharmacia Biotech, Uppsala, Sweden) using the fluorescent dye-labeled M13 forward or reverse primer (provided by the kit). The nucleotide sequences were obtained by electrophoresis on an ALF automatic sequencer (Perkin Elmer Co.).

### EXAMPLE 2

### Wounding treatment

Latex and leaf samples were collected from the rubber trees wounded with six nails above and along the slope of the tapping cut(Oh et al., 1999 J. Biol. Chem 274 : 17132-17138).

### Southern and northern blot analysis

Genomic DNA from leaf tissues of a mature *H. brasiliensis* RRIM600 was prepared as described(Oh et al., 1999 J. Biol. Chem 274 : 17132-17138). Genomic DNA (8-10 µg) was digested with the indicated restriction enzymes, size-fractionated by electrophoresis in 0.8 % (w/v) agarose gels, blotted, and cross-linked to Hybond-N nylon membrane (Amersham Life Science, Little Chalfont, England)) by UV radiation. For northern blot, twenty micrograms of total RNA were electrophoresed on a 1.2 % agarose-formaldehyde gel, transferred, and crossed-linked as for Southern blot. The probe for both hybridizations was a ³²P-labelled 700-bp PCR generated fragment (described above) of IPIHb cDNA. Hybridization was for 18 h at 55 °C for northern and 65 °C for Southern in 0.9 M sodium chloride, 50 mM sodium phosphate (pH 7.6), 5 mM EDTA, 0.4% (w/v) SDS, 5 X Denhardt's solution, and 200 µg salmon sperm DNA. The blot was washed at either 55°C for low stringency or 65 °C for high stringency twice in 0.5 X SSPE, 0.1% SDS, and twice in 0.1 X SSPE, 0.1% SDS. The membrane was exposed to an X-ray film overnight at -70°C.

### EXAMPLE 3

### Heterologous expression of IPIHb protein in E. coli

The IPIHb gene was cloned in the *EcoR*I*-Xho*I site of pGEX (an IPTG-inducible expression vector; Pharmarcia Biotech, Uppsala, Sweden) to construct pGEX-IPIHb. The *E. coli* XL1-Blue transformed with pGEX-IPIHb was grown to mid-stationary phase in Luria Broth (LB) containing 50 mg/l ampicillin at 30 °C with vigorous aeration. The cultures were induced by adding IPTG to a concentration of 0.1 mM and then incubated for another 6 h. All subsequent steps were carried out at 4 °C. The cells were harvested, washed with 0.1 M potassium phosphate (pH 7.4) by centrifugation (5000 X g, 10 min), and then disrupted by sonication. The sonication was carried out for six cycles of 30 s bursts followed by 30 s intervals in 1 ml of buffer containing 0.1 M potassium phosphate (pH 7.4). The homogenate was centrifuged at 10,000 X g for 10 min and the supernatant was subjected to SDS-PAGE with 12.5% slab gels according to the standard method of Laemmli(Laemmli, 1970 Nature 277 680-685). Proteins were stained by Coomassie Brilliant Blue R-250. The fusion protein was affinity purified using Glutathione Sepharose 4B affinity matrix (Pharmarcia Biotech, Uppsala, Sweden) according to the instructions of manufacturer.

### Assay of IPP isomerase activity

The assay is based on the acid lability of the allylic diphosphate described by Satterwhite(Satterwhite, 1985). The incubation mixture, total volume 50 *µ*l, contained 100 mM Tris-HCl, pH 7.5, 1.5 mM MgCl₂, 1.5 mM MnCl₂, 2 mM DTT, 25 mM KF, 20*µ*M [¹⁴C]IPP (55 mCi mmol⁻¹, Amersham), and suitable amounts of enzyme preparation. After 10 min incubation at 30 °C, 200 *µ*L methanol:HCl (4:1, v/v) and 0.1 *µ*L water were added and incubation was continued for 15 min at 37 °C. The allylic products were extracted with 2 X 200 ml toluene. The combined extracts were mixed with 4 ml Ready Solv HP scintillation cocktail (Beckman), and the radioactivity was determined using an LS 6500 liquid scintillation counter (Beckman).

### In vitro rubber biosynthesis assay

Washed rubber particles (WRP) were prepared by repeated centrifugation/flotation procedure as described(Cornish and Backhaus, 1990 Phytochemistry 27 : 3809-3813; Siler and Cornish, 1993 Phytochemistry 32 : 1097-1102). Rubber biosynthetic activity *in vitro* of the reaction mixtures was determined by the methods previously described(Cornish and Backhaus, 1990 Phytochemistry 27 : 3809-3813; Siler and Cornish, 1993 Phytochemistry 32 : 1097-1102). WRP was incubated in 50 *µ*l of reaction mixture containing100 mM Tris-HCl, pH 7.5, 80 *µ*M [¹⁴C]IPP (55 mCi mmol⁻¹, Amersham), 20 *µ*M FPP, 1 mM MgSO₄ and 1 mM DTT for 6 h at 25 °C. To investigate the role of IPP isomerase on rubber biosynthesis, FPP, an efficient initiating molecule for rubber biosynthesis, was removed from the reaction mixture, and, instead, suitable amounts of IPP isomerase were added to convert IPP to DMAPP that is one of the initiating molecules for rubber biosynthesis. For control experiment, 25 mM of EDTA was added to the reaction mixture to chelate Mg²⁺ ion necessary for rubber transferase activity. The resulting [¹⁴C]IPP-incorporated rubber was quantified by using either a filtration or a benzene extraction method. For filtration method, the reaction mixture was filtered through either 0.02 or 0.1 *µ*m anodisc membrane (Whatman), the filter was subjected to repeated washing with 1 M HCl and 95 % ethanol(Cornish and Backhaus, 1990 Phytochemistry 27 : 3809-3813), and the remaining-radioactivity on the washed filters was determined by a liquid scintillation counter (Beckman). For benzene extraction method, the reaction mixture was extracted three times with two volumes of benzene, the benzene extract was mixed with a Ready Solv HP scintillation cocktail (Beckman), and the radioactivity was determined by a liquid scintillation counter.

## Claims

1. A process for the production of rubber *in vitro* comprising the steps of providing a nucleic acid molecule encoding an isopentenyl diphosphate isomerase from *Hevea brasiliensis* (IPI Hb) having an amino acid sequence as depicted in Sequence ID No.: 2, transferring said nucleic acid molecule to a host cell to produce a transformed host cell, culturing said transformed host in a suitable medium for the production of IPI Hb and using said IPI Hb to catalyse the synthesis of rubber in vitro.

2. Process for the production of rubber *in vitro* comprising the steps of providing a nucleic acid molecule having a nucleic acid sequence as depicted in Sequence ID No. 1, transferring said nucleic acid molecule to a host cell to produce a transformed host cell, culturing said transformed host in suitable medium for the production of IPI Hb and using said IPI Hb to catalyse the synthesis of rubber *in vitro.*

3. A process for the production of rubber *in vivo* comprising the steps of providing a nucleic acid molecule encoding an isopentenyl diphosphate isomerase from *Hevea* brasiliensis (IPI Hb) having an amino acid sequence as depicted in Sequence ID No. 2, transferring said nucleic acid molecule to a host cell to produce a transformed host cell and culturing said transformed host in an environment that is suitable for the production of rubber *in vivo.*

4. A process as claimed in any claim 1 to 3, wherein said nucleic acid molecule is a vector.

5. A process claimed in claim 4, wherein said nucleic acid molecule is operatively linked to a promoter element.

6. A process as claimed in claim 5, wherein said promoter element is selected from the group consisting of bacterial promoter, plant promoter and virus promoter.

7. A process as claimed in claim 6, wherein said vector is transferred to and replicated in the host.

8. A process as claimed in any preceding claim 1 to 7, when said host is selected from a group consisting of plants, bacteria, yeasts and fungi.

## Patentansprüche

1. Verfahren zur Herstellung von Kautschuk *in vitro,* umfassend die Schritte: Bereitstellen eines Nukleinsäuremoleküls, das Isopentenyldiphosphatisomerase der *Hevea brasiliensis* (IPI Hb) kodiert, dessen Aminosäuresequenz wie in der Sequenz-ID Nr. 2 ist; Transferieren des Nukleinsäuremoleküls in eine Wirtszelle, so dass man eine transformierte Wirtszelle erhält; Kultivieren des transformierten Wirts in einem geeigneten Medium zur Herstellung von IPI Hb und Verwenden der IPI Hb zur Katalyse der *in vitro-*Synthese von Kautschuk.

2. Verfahren zur *in vitro*-Herstellung von Kautschuk, umfassend die Schritte: Bereitstellen eines Nukleinsäuremoleküls mit einer Nukleinsäuresequenz wie in der Sequenz-ID Nr. 1; Transferieren des Nukleinsäuremoleküls in eine Wirtszelle, so dass man eine transformierte Wirtszelle erhält; Kultivieren der transformierten Wirtszelle in einem geeigneten Medium zur Herstellung von IPI Hb und Verwenden der IPI Hb zur Katalyse der *in vitro*-Synthese von Kautschuk.

3. Verfahren zur *in vitro*-Herstellung von Kautschuk, umfassend die Schritte: Bereitstellen eines Nukleinsäuremoleküls, das Isopentenyldiphosphatisomerase von *Hevea brasiliensis* (IPI Hb) kodiert, dessen Aminosäuresequenz wie in der Sequenz-ID Nr. 2 ist; Transferieren des Nukleinsäuremoleküls in eine Wirtszelle, so dass man eine transformierte Wirtszelle erhält; und Kultivieren der transformierten Wirtszelle in einem Milieu, das sich für die *in vitro-*Herstellung von Kautschuk eignet.

4. Verfahren nach irgendeinem Anspruch 1 bis 3, wobei das Nukleinsäuremolekül ein Vektor ist.

5. Verfahren nach Anspruch 4, wobei das Nukleinsäuremolekül funktionell mit einem Promoterelement verknüpft ist.

6. Verfahren nach Anspruch 5, wobei das Promoterelement aus der Gruppe Bakterienpromoter, Pflanzenpromoter und Viruspromoter ausgewählt ist.

7. Verfahren nach Anspruch 6, wobei der Vektor in einen Wirt transferiert und repliziert wird.

8. Verfahren nach irgendeinem der vorhergehenden Ansprüche 1 bis 7, wobei der Wirt aus der Gruppe Pflanzen, Bakterien, Hefen und Pilze ausgewählt ist.

## Revendications

1. Procédé de production de caoutchouc *in vitro* comprenant les étapes consistant à fournir une molécule d'acide nucléique codant pour une isopentényl diphosphate isomérase d'*Hevea brasiliensis* (IPI Hb) ayant une séquence d'acides aminés telle que décrite dans Séquence ID n° 2, à transférer ladite molécule d'acide nucléique dans une cellule hôte pour produire une cellule hôte transformée, à cultiver ledit hôte transformé dans un milieu adapté pour la production d'IPI Hb et à utiliser ladite IPI Hb pour catalyser la synthèse de caoutchouc *in vitro.*

2. Procédé de production de caoutchouc *in vitro* comprenant les étapes consistant à fournir une molécule d'acide nucléique ayant une séquence d'acide nucléique telle que décrite dans séquence ID n° 1, à transférer ladite molécule d'acide nucléique dans une cellule hôte pour produire une cellule hôte transformée, à cultiver ledit hôte transformé dans un milieu adapté pour la production d'IPI Hb et à utiliser ladite IPI Hb pour catalyser la synthèse de caoutchouc *in vitro.*

3. Procédé de production de caoutchouc *in vivo* comprenant les étapes consistant à fournir une molécule d'acide nucléique codant pour une isopentényl diphosphate isomérase d'*Hevea brasiliensis* (IPI Hb) ayant une séquence d'acides aminés telle que décrite dans séquence ID n° 2, à transférer ladite molécule d'acide nucléique à une cellule hôte pour produire une cellule hôte transformée et à cultiver ledit hôte transformé dans un environnement qui est adapté pour la production de caoutchouc *in vivo.*

4. Procédé selon l'une quelconque des revendications 1 à 3, dans lequel ladite molécule d'acide nucléique est un vecteur.

5. Procédé selon la revendication 4, dans lequel ladite molécule d'acide nucléique est liée de manière fonctionnelle à un élément promoteur.

6. Procédé selon la revendication 5, dans lequel ledit élément promoteur est choisi dans le groupe constitué par un promoteur bactérien, un promoteur végétal et un promoteur viral.

7. Procédé selon la revendication 6, dans lequel ledit vecteur est transféré et répliqué dans l'hôte.

8. Procédé selon l'une quelconque des revendications précédentes 1 à 7, dans lequel ledit hôte est choisi dans un groupe constitué par des plantes, des bactéries, des levures et des champignons.
